(19) Europäisches Patentamt
European Patent Office
Office européen des brevets

(11) Publication number: 0 263 553 A2

# (12) EUROPEAN PATENT APPLICATION

(21) Application number: 87201841.1

(22) Date of filing: 24.09.87

(51) Int. Cl.4: C12N 15/00 , A01H 5/10 , A01N 43/16 , A01N 63/00 , //C05F11/08,(C12N1/20,C12R1:-41)

(30) Priority: 30.09.86 US 913805

(43) Date of publication of application: 13.04.88 Bulletin 88/15

(84) Designated Contracting States: AT BE CH DE ES FR GB GR IT LI LU NL SE

(71) Applicant: SHELL INTERNATIONALE RESEARCH MAATSCHAPPIJ B.V.
Carel van Bylandtlaan 30
NL-2596 HR Den Haag(NL)

(72) Inventor: Cunningham, Scott Daniel
5120 Shippee Lane
Stockton California 95212(US)

(74) Representative: Hunter, Keith Roger Ian et al
4 York Road
London SE1 7NA(GB)

## (54) Method of inhibiting gene induction.

(57) A method of inhibiting the induction of expression of a structural gene or other protein coding gene or DNA segment under the control of a nod gene promoter with certain 7-oxy flavones, flavonols, flavanones or isoflavones.

EP 0 263 553 A2

## METHOD OF INHIBITING GENE INDUCTION

The present invention relates to a method of inhibiting the induction of expression of a structural gene or other protein coding gene or DNA segment under the control of a nod gene promoter.

Nodulation (nod) genes are required by certain bacteria for the infection and subsequent nodulation of plant roots. The host specificity of the nod genes of Rhizobia (and Bradyrhizobia) species is known to include a family of plants, Leguminosae, including economically important crop species such as lentils, cowpeas, soy-beans, clover, alfalfa, beans, peanuts, garden peas and the like. Some Rhizobia infect other families of plants.

It is known that a certain factor X in plants can induce the expression of a nod gene. However, to utilise the capabilities of nod genes, it is desirable to increase the understanding of the genetics of nodulation and the inducers or inhibitors that control expression of the nodulation genes. Also, since it is difficult for superior nitrogen fixing bacteria to effect nodulation when in competition with indigenous strains in the rhizosphere of plants, it is desirable to find a method of inhibiting the induction of expression of the nod genes in indigenous species so as to allow newly introduced, superior nitrogen-fixing bacteria to form nodules.

The present invention provides a method of inhibiting or controlling the time of the induction of expression of a structural gene or other protein coding gene or DNA segment under the control of a nod gene promoter, which comprises administering to a host containing said nod gene promoter an amount, sufficient to inhibit the induction of expression of the gene or DNA segment, of a compound of Formula I, II or III

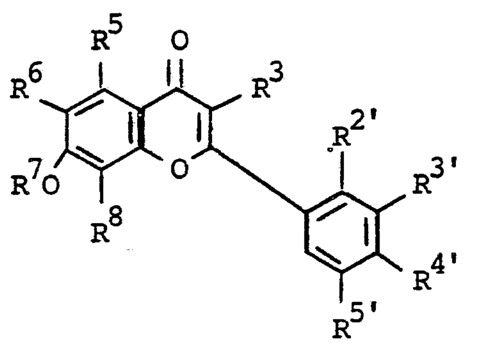

I flavones
flavonols $R^3$ = OH

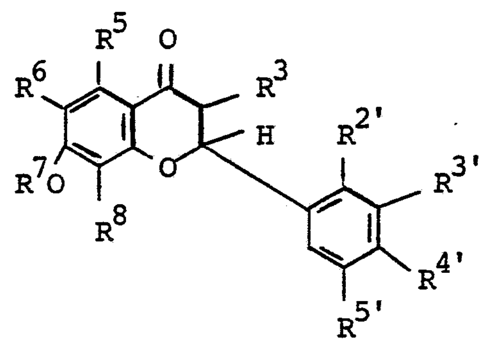

II flavanones
flavonols $R^3$ = OH

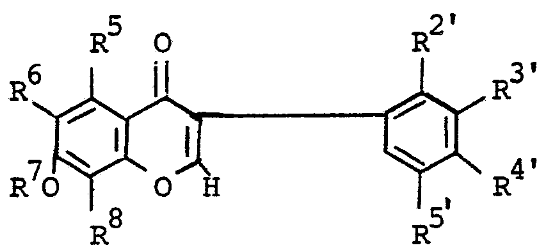

III isoflavones

wherein $R^7$ is H, methyl or ethyl; $R^{2'}$, $R^{3'}$, $R^{5'}$, $R^5$, $R^6$ and $R^8$ are each independently H, methyl, ethyl, methoxy, ethoxy or OH; $R^3$ is H or OH; $R^5$ is H, OH, methyl or ethyl; in Formula I when $R^3$ = H, then $R^{4'}$ is H, methyl, ethyl, methoxy or ethoxy or when $R^3$ = OH, then $R^{4'}$ is H, methyl, ethyl, hydroxy, methoxy or ethoxy; or in Formula II or III $R^{4'}$ is H, OH, methoxy or ethoxy.

The induction of nod ABC requires an inducer molecule, such as the plant extract factor X (now known to be luteolin apigenin, and with some Rhizobium, Eriodyctol or Naringenin) and the nod D gene product. Constitutive mutants of these genes are also known.

The inhibitor chemicals of Formula I, II and III have the opposite effect to factor X. They are useful in the study of the nod gene mechanism. Radiolabeled inhibitor chemicals are useful in the study of their interaction with the nod D gene product and to follow the fate of the inhibitors in the bacteria.

The compounds of Formula I, II and III are chemical inhibitors of the action of the natural plant products' action on induction of expression of the genes or DNA segments under control of a nod gene promoter resulting in the decreasing of expression of the genes, e.g. inhibiting expression of nodulation genes and inhibiting nodulation. At lower concentrations, the compounds of Formula I, II and III also increase the time required for nodules to form on plants.

By application of the method of the present invention it is possible to inhibit or delay the time of the induction of expression of a gene or DNA segment under control of a nod gene promoter in an easily controllable manner. The induced gene or DNA segment can be a natural or foreign sequence. Thus, the present invention provides a method for controlled regulation of efficient transcription of a (gene) sequence encoding for a desired protein.

Specific techniques for introducing structural genes into bacteria in order that the structural genes will be expressed in bacteria living symbiotically in the plant host are set forth in Long, S.R., T.T. Egelhoff, R.F. Fisher, T.W. Jacobs and J.T. Mulligan, Proceedings of the 6th International Symposium on Nitrogen Fixation, p.87-94 (1985) and Egelhoff, T.T. and S.R. Long, J. Bacteriology, 164(2), 591-599 (1985).

In another embodiment of the invention, genes coding for nodulation required products can be so engineered so as to not require the presence of an activating factor X to initiate the nodulation process.

The genes coding for the synthesis of these nodulation requirements can, if desired, be placed under the control of a second promoter, either a promoter indigenous to the Rhizobium or Bradyrhizobium host or other such as the lac promoter. This should allow nodulation to occur in plants by superior bacteria strains when the inhibitors of the invention turn-off indigenous strains.

This may also be accomplished by a simple, non-genetically engineered solution by a selection protocol for the bacteria which selects from the superior nitrogen fixing strain those members of that population which form nodules in the presence of the inhibitors disclosed in this patent.

Treating the rhizosphere of legume plants with the compounds of the invention, wild-type (naturally-occurring) strains of nodulating bacteria, e.g. Rhizobia, may be inhibited, applying to (inoculating) the rhizosphere an amount of at least one superior (more efficient) nitrogen-fixing Rhizobia strain and allowing said superior strain to nodulate, results in increasing the amount of nodulation by the more efficient nitrogen-fixing Rhizobia strain. Examples of superior nitrogen-fixing strains include commercially available strains (such as those sold by Nitragin Co.) as well as Bradyrhizobia japonicum USDA 110, C33 and the like available from the USDA Beltsville, Maryland and genetically engineered strains such as disclosed in European patent 130047 and the like. Such manipulation of the rhizosphere by inhibiting wild-type strains, then inoculating the rhizosphere with superior nitrogen-fixing strains and allowing said superior strain to nodulate, is useful for increasing the yield and protein content of plants, e.g. crops such as legumes, including alfalfa, soybeans, peanuts and the like.

Thus, superior nitrogen-fixing bacteria can be induced to fix-nitrogen and in some cases also assist plants to exhibit resistance against invasion by foreign organisms, such as pathogens, nematodes, insects, and the like, by providing polypeptides that act either alone or with their products or other agents as toxins against such pests (e.g. Bacillus thuringiensis endotoxin protein). These agents may also protect against chemical imbalances or excess toxic chemicals, and the like. The agents can control amino acid levels in the plants, and generally increase or decrease nutrient levels. Further, the superior bacteria can contain genes encoding agents having growth control activity over the plants or portions thereof (e.g., seeds, roots, shoots, leaves, fruit and the like). Indeed, selective herbicidal proteins or proteins useful in herbicide detoxification (such as against residual atrazine) could also be produced.

Genes encoding polypeptides capable of inducing herbicidal resistance in plants have previously been cloned and introduced into plants. These include the bacterial genes encoding for chlorsulfuron and sulfometuron resistance, as well as the glyphosate resistance gene from Salmonella. If desired, these and various additional genes may be provided in a polycistronic form. The particular structural gene or other protein coding gene or DNA segment thereof naturally or synthetically produced which encodes for the effective desired structural sequence of a protein or polypeptide, inserted as an agent is not critical to this aspect of the present invention, and any polypeptide or protein of interest may be prepared employing these constructions as described herein.

The invention also includes a method for combating undesirable plant growth at a locus which comprises applying to the plant a nodulation-inhibiting amount of a compound of Formula I, II or III of the invention. Thus, undesirable plants, e.g. legume weeds, are killed or damaged by their inability to obtain sufficient fixed nitrogen to continue life or growth.

The bacteria of the genus Rhizobia useful in this invention include R. trifolii, R . japonicum, R. meliloti, phascoli, leguminosarum and (cowpea miscellany) and the like, including genetically modified bacteria of this genus or those bacteria having the characteristic thereof to form nodules on plants. Not every strain of Rhizobia is susceptible to inhibition by each and every compound of the invention. However, it is a routine matter to conduct the Nod-gene inhibition test on any species of interest. For example, in Rhizobial leguminosarum strains the compound Eriodyctol and Naringenin may act similar to Luteolin and induce nod genes ABC.

The compounds for use in this invention are certain compounds of the class of flavones, flavonols, flavanones and isoflavanones, which are generally known in the art and are natural materials extracts, e.g. from plants available from commercial sources or are produced by synthetic techniques. For example, appropriately substituted flavones of Formula I are prepared by rearrangement of 2-aroyloxyacetophenones to 2-hydroxy derivatives of dibenzoylmethanes followed by cyclization by, e.g., hydrogen bromide in glacial acetic acid, where the substitution pattern in the flavones are determined by the substitution patterns of the original phenyl groups.

Non-limiting representative compounds for use in the invention include:

Formula I

| Flavones | $R^3$ | $R^5$ | $R^6$ | $R^7$ | $R^8$ | $R^{3'}$ | $R^{4'}$ |
|---|---|---|---|---|---|---|---|
| Acacetin | H | OH | H | H | H | H | $OCH_3$ |
| Chrysin | H | OH | H | H | H | H | H |
| Eupatorin | H | OH | $OCH_3$ | $CH_3$ | H | OH | $OCH_3$ |
| 7-OH flavone | H | H | H | H | H | H | H |
| 7-OH,5-methyl flavone | H | $CH_3$ | H | H | H | H | H |
| Norwagonine | H | OH | H | H | OH | H | H |
| Diosmetin | H | OH | H | H | H | OH | $OCH_3$ |
| **Flavonols** | | | | | | | |
| 3'4'-Dimethoxy-3,5,7-trihydroxy | OH | OH | H | H | H | $OCH_3$ | $OCH_3$ |
| Fisetin | OH | H | H | H | H | OH | OH |
| Galangin | OH | OH | H | H | H | H | H |
| Geraldol | OH | H | H | H | H | $OCH_3$ | OH |
| Isorhamnetin | OH | OH | H | H | H | $OCH_3$ | OH |
| Kaempferol | OH | OH | H | H | H | H | OH |
| Rhamnetin | OH | OH | H | $CH_3$ | H | OH | OH |
| Tamarixetin | OH | OH | H | H | H | OH | $OCH_3$ |

Formula II

| Flavanone | | | | | | | |
|---|---|---|---|---|---|---|---|
| Naringenin | H | OH | H | H | H | H | OH |
| Eriodyctol | H | OH | H | H | H | OH | OH |

Formula III

| Isoflavanone | | | | | | | |
|---|---|---|---|---|---|---|---|
| Biochanin A | H | OH | H | H | H | H | $OCH_3$ |

In one Embodiment of the invention, the compound is acacetin, chrysin, 5-methyl-7-hydroxyflavone, naringenin, rhamnetin, tamarixeten and the like. Preferably, the compound is acacetin or chrysin.

For application, a compound of Formula I, II or III ordinarily is applied most effectively by formulating it with a suitable inert carrier or surface-active agent, or both. The invention, therefore, also includes compositions comprising an inert carrier or surface-active agent, or both, and as active ingredient at least one compound of Formula I, II or III.

The term "carrier" as used herein means an inert solid or liquid material, which may be inorganic or organic and of synthetic or natural origin, with which the active compound is mixed or formulated to facilitate its application to the plant, seed, soil or other object to be treated, or its storage, transport and/or handling. Any of the materials customarily employed in formulating pesticides, herbicides, or fungicides -i.e., horticulturally acceptable carriers - are suitable.

Suitable solid carriers are natural and synthetic clays and silicates, for example, natural silicas such as diatomaceous earths; magnesium silicates, for example, talcs; magnesium aluminum silicates, for example attapulgites and vermiculites; aluminum silicates, for example, kaolinites, montmorillonites and micas; calcium carbonate; calcium sulfate; synthetic hydrated silicon oxides and synthetic calcium or aluminum silicates; elements such as, for example, carbon and sulphur; natural and synthetic resins such as, for example, coumarone resins, polyvinyl chloride and styrene polymers and copolymers; bitumen; waxes such as, for example, beeswax, paraffin wax, and chlorinated mineral waxes; solid fertilizers, for example, superphosphates; and ground, naturally-occurring, fibrous materials, such as ground corncobs.

Examples of suitable liquid carriers are water, alcohols such as isopropyl alcohol and glycols; ketones such as acetone, methyl ethyl ketone, methyl isobutyl ketone and cyclohexanone; ethers such as cellosolves; aromatic hydrocarbons such as benzene, toluene and xylene; petroleum fractions such as kerosene, light mineral oils; chlorinated hydrocarbons such as carbon tetrachloride, perchloroethylene and trichloromethane. Also suitable are liquefied, normally vaporous and gaseous compounds. Mixtures of different liquids are often suitable.

The surface-active agent may be an emulsifying agent or a dispersing agent or a wetting agent; it may be nonionic or ionic. Any of the surface-active agents usually applied in formulating herbicides or insecticides may be used. Examples of suitable surface-active agents are the sodium and calcium salts of polyacrylic acids and lignin sulfonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols or alkyl phenols, for example, p-octylphenol or p-octylcresol, with ethylene oxide and/or propylene oxide; sulfates or sulfonates of these condensation products, alkali or alkaline earth metal salts, preferably sodium salts, of sulfuric or sulfonic acid esters containing at least 10 carbon atoms in the molecule, for example, sodium lauryl sulfate, sodium secondary alkyl sulfates, sodium salts of sulfonated castor oil, and sodium alkylaryl sulfonates such as sodium dodecylbenzene sulfonate; and polymers of ethylene oxide and copolymers of ethylene oxide and propylene oxides.

The compositions of the invention may be prepared as wettable powders, dusts, granules, solutions, emulsifiable concentrates, emulsions, suspension concentrates and aerosols. Wettable powders are usually compounded to contain 25 to 75% by weight of active compound and usually contain, in addition to the solid carrier, 3-10% by weight of a dispersing agent, 2-15% of a surface-active agent and, where necessary, 0-10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5-10% by weight of the active compound. Granules are usually prepared to have a size between 10 and 100 BS mesh (1.676-0.152 mm), and may be manufactured by agglomeration or impregnation techniques. Generally, granules will contain 0.5-25% by weight of the active compound, 0-1% by weight of additives such as stabilizers, slow release modifiers and binding agents. Emulsifiable concentrates usually contain, in addition to the solvent and, when necessary, cosolvent, 10-50% weight per volume of the active compound, 2-20% weight per volume emulsifiers and 0-20% weight per volume of appropriate additives such as stabilizers, penetrants and corrosion inhibitors. Suspension concentrates are compounded so as to obtain a stable, non-sedimenting, flowable product and usually contain 10-75% weight of the active compound, 0.5-5% weight of dispersing agents, 1-5% of surface-active agent, 0.1-10% weight of suspending agents, such as defoamers, corrosion inhibitors, stabilizers, penetrants and stickers, and as carrier, water or an organic liquid in which the active compound is substantially insoluble; certain organic solids or inorganic salts may be dissolved in the carrier to assist in preventing sedimentation or as antifreeze agents for water.

Of particular interest in current practice are waterdisperable granular formulations. These are in the form of dry, hard granules that are essentially dust-free, and are resistant to attrition on handling, thus minimising the formation of dust. On contact with water, the granules readily distintergrate to form stable suspensions of the particles of active material. Such formulations contain 90% or (up to 95%) more by weight of finely divided active material, 3-7% by weight of a blend of surfactants, which act as wetting, dispersing, suspending and binding agents, and may contain up to 3% by weight of a finely divided carrier, which acts as a resuspending agent.

Aqueous dispersions and emulsions, for example, compositions obtained by diluting a wettable powder or a concentrate according to the invention with water, also lie within the scope of the present invention. The said emulsions may be of the water-in-oil or of the oil-in-water type, and may have thick, mayonnaise-like consistency.

It is evident from the foregoing that this invention contemplates compositions containing as little as about 0.5% by weight to as much as about 95% by weight of a compound of Formula I, II or III as the active ingredient.

The compositions of the invention may also contain other ingredients, for example, other compounds possessing pesticidal, especially insecticidal, acaricidal, herbicidal or fungicidal properties, fertilizers, mulching agents as are appropriate to the intended purpose.

The desired effect on plants is effected by applying a compound of Formula I, II or III, ordinarily in a composition of one of the aforementioned types, to soil (rhizosphere) in which the seeds of the plants will be planted or are present, or to the foliage of the plants. The compounds employed in the method of the invention are transported by a plant and thus readily reach the unicellular microorganism (bacteria) living symbiotically in the plants when applied directly to plant, including plant parts, such as leaves or roots. Compounds can also be applied directly to seeds in the presence of bacteria.

The active compound, of course, is applied in an amount sufficient to exert the desired action. The amount of the compound of Formula I, II or III to be used in plants will naturally depend on the condition of the plants, the degree of activity desired, the formulation used, the mode of application, the climate, the season of the year, and other variables.

The inhibitor chemicals of Formula I, II or III of the invention are used at concentrations similar to that of the inducer factor X (luteolin) and the like as well as for other inducible systems, such as the lactose operon and the arabinose operon, although usually several times lower than the latter two systems. For example, the compound is used in an amount sufficient to produce a concentration in the environment in contact with the host of from about 0.01 to about 10.0 $\mu$M and, preferably, from about 0.1 to about 1.0 $\mu$m.

The invention also includes a seed formulation comprising a plant seed, a superior nitrogen-fixing, nodulating bacteria strain and at least one compound of Formula I, II or III of the invention and optionally a granular inert carrier material. The granular inert carrier is preferably a hydrocarbonaceous carrier which will provide a habitat for the initial propagation of the desired superior nitrogen-fixing, nodulating bacteria strain.

Illustrative Embodiments

The following Embodiments are offered to illustrate the invention.

Nod-gene Inhibition

Sterile alfalfa seeds (cv. Moapa 69) were planted onto the surface of agar poured on a slant in a test tube. The test solution used to make agar was 0 nitrogen Hoaglands solution. Ten replicates of each concentration of test solution of each compound were so planted. The test tubes were inoculated with *Rhizobium meliloti* 1021 3 days after planting. These were placed in the greenhouse and scored for number of nodules/tube and number of tubes with nodules at days 24 and 28. The results are set forth in Table 1 below.

Table 1. Alfalfa/Analog Test Tube Trails

| Treatment | nodules/tube | |
|---|---|---|
| | day 24 | day 28 |
| Control | 3.4 | 4.4 |
| Luteolin (.07 μM) | 1.8 | 3.5 |
| Luteolin (.7 μM) | 1.7 | 3.0 |
| Luteolin (70 μM) | 1.2 | 5.2 |
| Galangin (.07 μM) | 1.2 | 2.5 |
| Galangin (.7 μM) | 0.1 | 0.3 |
| Galangin (70 μM) | 0.1 | 1.6 |
| Acacetin (.07 μM) | 1.9 | 2.7 |
| Acacetin (.7 μM) | 1.2 | 4.9 |
| Acacetin (70 μM) | 1.0 | 2.2 |
| Chrysin (0.7 μM) | 2.0 | 3.9 |
| Chrysin (.7 μM) | 1.9 | 4.2 |
| Chrysin (70 μM) | 0 | 0.8 |
| 7-OH,5-$CH_3$ (0.7 μM) | 1.6 | 3.1 |
| 7-OH,5-$CH_3$ (.7 μM) | 1.3 | 3.0 |
| 7-OH,5-$CH_3$ (70 μM) | 0 | 0 |

Yield Increase

Alfalfa seeds (cv. Moapa 69) are planted into soil containing competitive wild-type nitrogen-fixing bacteria held in pots. The test solution is made up of inhibitors as in the previous Embodiment. The replicates of each concentration of test solution of each test compound are so planted. The planted pots are inoculated with a selected superior nitrogen-fixing, nodulating bacteria *Rhizobia meliloti* not normally competitive against the wild type. The plants are grown to harvest and the weight of harvested above ground plant parts are as determined.

Representative compounds employed in the method according to the invention, acacetin and chrysin, inhibit nodulation of the indigenous wild-type bacteria allowing the superior bacteria *R. meliloti* to establish itself and nodulate the alfalfa plants resulting in an increase in the crop yield.

## Claims

1. A method of inhibiting or controlling the time of the induction of expression of a structural gene or other protein coding gene or DNA segment under the control of a <u>nod</u> gene promoter, which comprises administering to a host containing said <u>nod</u> gene promotor an amount sufficient to inhibit the induction of expression of the gene or segment of a compound of Formula I, II or III

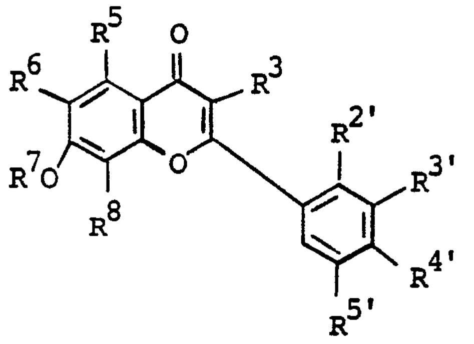

I flavones
flavonols $R^3$ = OH

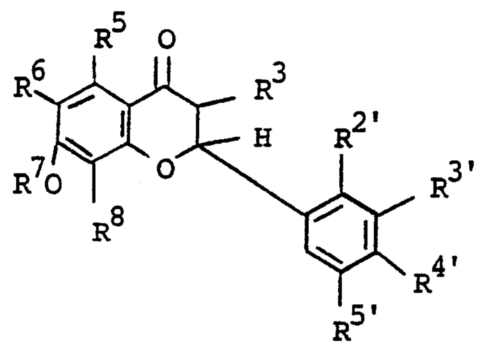

II flavanones
flavonols $R^3$ = OH

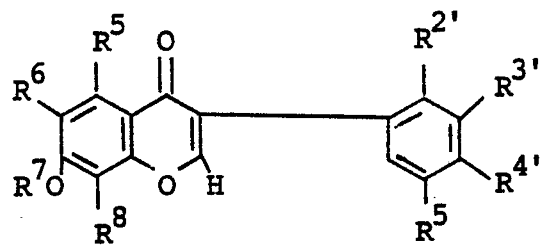

III isoflavones

wherein $R^7$ is H, methyl or ethyl, $R^{2'}$, $R^{3'}$, $R^{5'}$, $R^5$, $R^6$ and $R^8$ are each independently H, methyl, ethyl, methoxy, ethoxy or OH; $R^3$ is H or OH; $R^5$ is H, OH, methyl or ethyl; in Formula I when $R^3$ = H, $R^{4'}$ is H, methyl, ethyl, methoxy or ethoxy or in Formula I when $R^3$ = OH, $R^{4'}$ is H, methyl, ethyl, hydroxy, methoxy or ethoxy; or in Formula II or III $R^{4'}$ is H, OH, methoxy or ethoxy.

2. A method of increasing the time required for nodules to form on plants comprises treating the rhizosphere of the plants with an effective amount of at least one compound of Formula I, II or III as defined in claim 1.

3. A method of decreasing nodulation on plants which comprises treating the rhizosphere of the plants with a nodulation-inhibiting amount of at least one compound of Formula I, II or III as defined in claim 1.

4. A method for infecting plants with a superior nitrogen-fixing nodulating bacteria strain which comprises treating the rhizosphere with a nodulation-inhibiting amount of at least one compound of Formula I, II or III as defined in claim 1 to inhibit the nodulation of wild-type strains of nodulating bacteria, then introducing the superior nitrogen-fixing, nodulating bacteria strain and allowing said superior strain to nodulate.

5. A method for increasing the yield of plants comprises treating the rhizosphere of the plants with an amount of a compound of Formula I, II or III as defined in claim 1 sufficient to inhibit nodulation of wild-type bacteria, then introducing a superior nitrogen-fixing, nodulating bacteria strain and allowing said superior strain to nodulate.

6. A method of combating undesirable plant growth at a locus comprises applying to the plant or the locus a nodulation inhibiting amount of a compound of Formula I, II or III as defined in claim 1.

7. A composition for controlling the induction of expression of a structural gene or other protein coding gene or DNA segment under the control of a nod gene promoter comprises a compound of Formula I, II or III as defined in claim 1 and at least one agronomically-acceptable carrier or surface-active agent.

8. A seed formulation comprising a plant seed, a superior nitrogen-fixing, nodulating bacteria strain and at least one compound of Formula I, II or III as defined in claim 1.

9. A method as claimed in any one of claims 2 to 5 wherein the plants are selected from alfalfa, soybeans, peanuts, common beans, cowpeas, lentil, clover, and like.

10. A method as claimed in any one of claims 1 to 6 and 9 in which the compound of Formula I, II or III is a flavone or flavonol of Formula I wherein

| Flavones | $R^3$ | $R^5$ | $R^6$ | $R^7$ | $R^8$ | $R^{3'}$ | $R^{4'}$ |
|---|---|---|---|---|---|---|---|
| Acacetin | H | OH | H | H | H | H | $OCH_3$ |
| Chrysin | H | OH | H | H | H | H | H |
| Eupatorin | H | OH | $OCH_3$ | $CH_3$ | H | OH | $OCH_3$ |
| 7-OH flavone | H | H | H | H | H | H | H |
| 7-OH,5-methyl flavone | H | $CH_3$ | H | H | H | H | H |
| Norwagonine | H | OH | H | H | OH | H | H |
| Diosmetin | H | OH | H | H | H | OH | $OCH_3$ |
| **Flavonols** | | | | | | | |
| 3'4'-Dimethoxy-3,5,7-trihydroxy | OH | OH | H | H | H | $OCH_3$ | $OCH_3$ |
| Fisetin | OH | H | H | H | H | OH | OH |
| Galangin | OH | OH | H | H | H | H | H |
| Geraldol | OH | H | H | H | H | $OCH_3$ | OH |
| Isorhamnetin | OH | OH | H | H | H | $OCH_3$ | OH |
| Kaempferol | OH | OH | H | H | H | H | OH |
| Rhamnetin | OH | OH | H | $CH_3$ | H | OH | OH |
| Tamarixetin | OH | OH | H | H | H | OH | $OCH_3$ |

or a flavanone of Formula II wherein

| Flavanone | | | | | | | |
|---|---|---|---|---|---|---|---|
| Naringenin | H | OH | H | H | H | H | OH |
| Eriodyctol | H | OH | H | H | H | OH | OH |

or an isoflavanone of Formula III where $R^3$, $R^6$, $R^7$, $R^8$ and $R^{3'}$are H and $R^5$ is OH and R $^{4'}$ is methoxy.

11. A method as claimed in claim 10 wherein the compound of Formula I, II or III is acacetin, chrysin, 5-methyl-7-hydroxy-flavone, naringenin, rhamnetin or tamarixeten.

12. A method as claimed in claim 11 wherein the compound of Formula I, II or III is acacetin or chrysin.

13. A method according to claim 1 wherein the host is R. meliloti , R. trifolii or R. japonicum, R. leguminosarum, Rhizobium (cowpea misc), Bradyrhizobium japonicum.

14. A method according to claim 1 wherein the host is a unicellular organism genetically engineered to contain said structural gene or DNA segemnt.

15. A method according to claim 1 wherein the gene or DNA segment is for (a) a pesticide or (b) herbicide detoxification.

16. A method according to claim 1 wherein the amount of said compound is sufficient to produce a concentration in the environment in contact with the host of 0.01 to 10.0 μM.

17. A method according to claim 16 wherein the amount of said compound is sufficient to produce a concentration in the environment in contact with the host of about 0.1 to 1.0 μM.

18. A method according to claim 4 or 5 wherein the superior nitrogen-fixing nodulating strain bacteria is Bradyrhizobia japonicum USDA110, C33 or a genetically-engineered superior strain.

19. A seed formulation according to claim 8 additionally containing an inert carrier.